# EUROPEAN PATENT APPLICATION

(11) **EP 2 322 181 A2**
(43) Date of publication of application: **18.05.2011**
(21) Application number: 09812597.4
(22) Date of filing: 25.08.2009
(51) Int. Cl.: A61K 31/522

(54) **A PENTOXIFILIN-BASED DERMATOLOGICAL PHARMACEUTICAL COMPOSITION, FOR TOPICAL APPLICATION, IN CREAM, GEL, OINTMENT, SOLUTION, EMULSION, LIPOSOME AND MICROCAPSULE FORM**

(30) Priority: 12.09.2008 CL 27302008
(71) Applicant: Figueroa Lizama, Patricio Roberto, Santiago 758-0206 (CL)
(72) Inventor: Figueroa Lizama, Patricio Roberto, Santiago 758-0206 (CL)
(74) Representative: Andrews, Martyn Peter
(86) International application number: PCT/CL2009/000011
(87) International publication number: WO 2010/028515

(57) **Abstract**

A pharmaceutical dermatological composition based on Pentoxifylline, to be applied on the skin in the form of a cream, gel, unguent, solution, in an emulsion, in liposomes and in microcapsules, being selective for the vascular disorders of rosacea and disorders with photosensitivity, reducing the blood vessels and the reactions of reddening of the skin, **characterised in that** said composition consists of: from 1 % to 99% of Pentoxifylline, from 0.1 % to 80% of base cream and from 0.1% to 75% of liposomes.

## Description

### Technical field

The invention for which a patent is being requested refers to a pharmaceutical dermatological composition based on pentoxifylline, to be applied on the skin in the form of a cream, gel, unguent, solution, in an emulsion, in liposomes and in microcapsules, being selective for the vascular disorders of rosacea and disorders with photosensitivity, reducing the blood vessels and the reactions of reddening of the skin.

### Background art

At present topical metronidazole exists for rosacea-related vascular disorders, whose composition is:
- Gel: every 100 gr contains Metronidazole 0.75 gr., Vehicle c.s.
- Dermal cream: every 100 gr contains Metronidazole 0.75 gr., Vehicles c.s.
- Forte cream: every 100 gr contains Metronidazole 1 gr, Vehicles c.s.

Metronidazole is good for the treatment of the inflamed papules and pustules, and in rosaceous erythema. Topical treatment and reduction of the clinical symptomatology of the rosacea in moderate or severe form (papules, pustules and erythema).

According to medical instructions, the customary dose is one application as a fine film with soft massages twice a day of the Geloderm Forte cream for a period of eight (8) weeks, although the therapeutic effects start to appear by the third week. The affected area must be cleaned before applying the medication.

Collateral effects of the metronizole

Reactions of hypersensitivity may occur. The contact with mucosae may cause local irritation, burning, itching, reddening, weeping, and dry skin. Not to be discarded are the effects reported for the oral administration of metronidazole, although the systemic absorption is minimum and the possibility of said effects is very low.

Pentoxifylline is used in peripheral vascular diseases as a coadyuvant of surgery for the treatment of the intermittent claudicating related to chronic occlusive arterial disease of the arms and legs, cerebral vascular insufficiency.

Sepsis is the intoxication of the blood by an infectious agent that reaches the blood stream. In general, it is fatal when it appears in newborn babies, particularly in those that are premature (before 37 weeks). The development of the resistance to antibiotics has made it more difficult to treat sepsis in an effective manner. Pentoxifylline is an anti-inflammatory medication that can reduce the sepsis and the complications originating in the sepsis. The revision found some proof that pentoxifylline in combination with antibiotics reduces mortality as a result of sepsis in newborn babies without causing adverse effects. More research needs to be carried out on pentoxifylline and other anti-inflammatory medicines that can be used for sepsis in newborn babies.

The principal objective of this invention is a composition based on pentoxifylline for medicinal dermal use; in any of the form, said adequate composition or mixture of the pentoxifylline with a solution or base cream, solves and actually improves the treatment of Rosacea and of disorders with photosensitivity, as well as vascular alterations of the skin.

Photosensitivity refers to cutaneous hyper-reactivity to luminous radiations that is observed in certain persons exposed to the action of exterior or endogenous sensitizing factors.

### Disclosure of the invention

In view of the above, some preferred executions of this invention are given; there is the possibility of varying the viscosity of the product that will be obtained.

Example 1:

| Formula: | |
|---|---|
| Pentoxifylline | 5% |
| Base Cream | 70% |
| Liposomes | 25% |

Example 2:

| Formula: | |
|---|---|
| Pentoxifylline | 5% |
| Base cream | 95% |

Example 3:

| Formula: | |
|---|---|
| Pentoxifylline | 1% |
| Base Cream | 90% |
| Liposomes | 9% |

Example 4:

| Formula: | |
|---|---|
| Pentoxifylline | 10% |
| Base Cream | 80% |
| Liposomes | 10% |

The composition to obtain a dermatological cream in the form of a liquid, gel, unguent, solution, in an emulsion, in liposomes and in microcapsules, based on pentoxifylline, for application to the skin, is the following: from 1 % to 99% of pentoxifylline, from 0.1 % to 80% of base cream and from 0.1 % to 75% of liposomes.

## Claims

1. A pharmaceutical dermatological composition based on pentoxifylline, to be applied on the skin in the form of a cream, gel, unguent, solution, in an emulsion, in liposomes and in microcapsules, being selective for the vascular disorders of rosacea and disorders with photosensitivity, reducing the blood vessels and the reactions of reddening of the skin, **CHARACTERIZED in that** said composition consists of: from 1 % to 99% of pentoxifylline, from 0.1 % to 80% of base cream and from 0.1 % to 65% of liposomes.

2. A pharmaceutical dermatological composition, according to claim 1, **characterised in that** the composition contains 90% of pentoxifylline, 5% of a base cream and 5% of liposomes.

3. A pharmaceutical dermatological composition, according to claim 1, **characterised in that** it contains 95% pentoxifylline and 5% base cream.

4. A pharmaceutical dermatological composition, according to claim 1, **characterised in that** the composition contains 80% pentoxifylline, 10% base cream and 10% liposomes.

5. A pharmaceutical dermatological composition, according to claims 1, 2, 3 and 4, **characterised in that** the composition is used for medicinal dermal treatment of Rosacea and the disorders with photosensitivity, as well as for vascular alterations of the skin.
